Europäisches Patentamt

European Patent Office

Office européen des brevets

(19)

(11) EP 0 596 923 B1

(12) **EUROPÄISCHE PATENTSCHRIFT**

(45) Veröffentlichungstag und Bekanntmachung des Hinweises auf die Patenterteilung:
**27.12.1996 Patentblatt 1996/52**

(21) Anmeldenummer: **92915504.2**

(22) Anmeldetag: **10.07.1992**

(51) Int Cl.$^6$: **C07D 307/92**

(86) Internationale Anmeldenummer:
**PCT/EP92/01565**

(87) Internationale Veröffentlichungsnummer:
**WO 93/02073 (04.02.1993 Gazette 1993/04)**

(54) **VERFAHREN ZUR HERSTELLUNG VON 8,12-OXIDO-13,14,15,16-TETRANORLABDAN**

METHOD OF PREPARING 8,12-OXIDO-13,14,15,16-TETRANORLABDANE

PROCEDE DE PREPARATION DE 8,12-OXIDO-13,14,15,16-TETRANORLABDANE

(84) Benannte Vertragsstaaten:
**CH DE ES FR GB IT LI NL**

(30) Priorität: **18.07.1991 DE 4123767**

(43) Veröffentlichungstag der Anmeldung:
**18.05.1994 Patentblatt 1994/20**

(73) Patentinhaber: **Henkel Kommanditgesellschaft auf Aktien**
**40191 Düsseldorf (DE)**

(72) Erfinder: **GERKE, Thomas**
**D-4040 Neuss (DE)**

(56) Entgegenhaltungen:
**WO-A-90/12793**

- **Chemical Abstracts, Band 105, Nr. 15, 13. Oktober 1986, (Columbus, Ohio, US) siehe Seite 703, Zusammenfassung 134193k, & JP, A, 61033184 (TAKASAGO PERFUMERY) 24-07-1986**

**Beschreibung**

Die Erfindung betrifft ein Verfahren zur Herstellung von Stereoisomerengemischen von 8,12-Oxido-13,14,15,16-tetranorlabdan durch cyclisierende Dehydratisierung von 8,12-Dihydroxy-13,14,15,16-tetranorlabdan in Gegenwart von säurebeladenem Aluminiumoxid.

8α,12-Oxido-13,14,15,16-tetranorlabdan, fortan als Ambroxan bezeichnet, ist ein begehrter Riechstoff mit ausgeprägtem Ambrageruch. Synthetisch ist Ambroxan in einer mehrstufigen Synthesesequenz ausgehend vom Sclareol zugänglich. So läßt sich z.B. das geruchlose Diol 8α,12-Dihydroxy-13,14,15,16-tetranorlabdan durch oxidativen Seitenkettenabbau von Sclareol gemäß US 30 50 532 und anschließende Reduktion des gebildeten Lactons herstellen. Durch dehydratisierende Cyclisierung dieses Diols gelangt man zum Ambroxan. Für diese Cyclisierung sind in der Literatur zahlreiche saure Katalysatoren beschrieben worden. Dabei ist insbesondere die deutsche Offenlegungsschrift DE 39 12 318 AI hervorzuheben, die ein Verfahren zur stereoselektiven Herstellung von Ambroxan unter Verwendung von säurebeladenem Aluminiumoxid beschreibt.

Die Herstellung von Stereoisomeren des Ambroxans ist jedoch ohne Ausbeuteeinbußen nicht ohne weiteres möglich. So haben P.F.Vlad und N.D.Ungur die Herstellung des 8-epi- und des 9-epi-Isomers mit der des Ambroxans durch Ringschluß der entsprechenden 1,4-Diol-Vorstufen mit Dimethylsulfoxid/Chlortrimethylsilan untersucht. Sie fanden, daß die Ausbeute bei der Cyclisierung in hohem Maße von der Stereochemie des Eduktes abhängig ist: Während Ambroxan und sein 9-epi-Isomer mit 85%- bzw. 90%-iger Ausbeute erhalten wurden, fiel das 8-epi-Isomer nur mit 46%-iger Ausbeute an.

Es ist ferner bekannt, daß auch Gemische der Stereoisomeren von Ambroxan einen ausgeprägten Ambrageruch aufweisen. So beschreibt die DE 32 40 054 A1 ein Verfahren zur Herstellung eines Gemisches von Stereoisomeren des Ambroxans. Bei diesem Verfahren wird Homofarnesylsäure in Gegenwart von Titantetrachlorid und/oder bestimmter Zinnverbindungen zu Norambreinolid cyclisiert, dieses in ein 1,4-Diol überführt und dieses zu 8,12-Oxido-13,14,15,16-tetranorlabdan cyclisiert. Gemäß dem Ausführungsbeispiel der DE 32 40 054 A1 beträgt die Ausbeute bei der letztgenannten Cyclisierung lediglich 65%.

Die Aufgabe der vorliegenden Erfindung bestand deshalb darin, ein Verfahren zu entwickeln, bei dem Stereoisomerengemische von 8,12-Dihydroxy-13,14,15,16-tetranorlabdan mit hoher Ausbeute in ein Stereoisomerengemisch von 8,12-Oxido-13,14,15,16-tetranorlabdan überführt werden.

Es wurde nun überraschend gefunden, daß sich Stereoisomerengemische von 8,12-Dihydroxy-13,14,15,16-tetranorlabdan mit einer Ausbeute von wenigstens 95% in Stereoisomerengemische von 8,12-Oxido-13,14,15,16-tetranorlabdan überführen lassen, wenn die Cyclisierung in Gegenwart von säurebeladenem Aluminiumoxid durchgeführt wird.

Gegenstand der Erfindung ist dementsprechend ein Verfahren zur Herstellung von Stereoisomerengemischen von 8,12-Oxido-13,14,15,16-tetranorlabdan durch cyclisierende Dehydratisierung von Stereoisomerengemischen von 8,12-Dihydroxy-13,14,15,16-tetranorlabdan mit säurebeladenem Aluminiumoxid.

Das erfindungsgemäße Verfahren wird bei Temperaturen von 120 bis 180 °C, vorzugsweise bei 140 bis 160 °C durchgeführt. Die Cyclisierung kann ohne Lösungsmittel oder vorzugsweise in inerten Lösungsmitteln, z.B. Toluol, Xylol oder Methylcyclohexan durchgeführt werden. Dabei lassen sich sowohl geringe Mengen an Wasser, die auf dem säurebeladenen Aluminiumoxid absorbiert sind, als auch das bei der Cyclisierung entstehende Wasser in einfacher Weise azeotrop abdestillieren.

Das säurebeladene Aluminiumoxid wird - bezogen auf das 8,12-Dihydroxy-13,14,15,16-tetranorlabdan - in einer Menge von 40 bis 100 Gew.-%, vorzugsweise 60 bis 80 Gew.-% eingesetzt.

Zur Beladung des Aluminiumoxids geeignete Säuren lassen sich in einem einfachen Test ermitteln: Man mißt den pH-Wert einer 5 gew.%-igen wäßrigen Dispersion von Aluminiumoxid, an dessen Oberfläche eine Säure adsorptiv gebunden ist. Liegt dieser pH-Wert zwischen 3 und 5,5, so ist die entsprechende Säure zur Beladung von Aluminiumoxid geeignet. Vorzugsweise eignen sich die Säuren, bei denen in diesem Test ein pH-Wert im Bereich von 4 bis 5 gefunden wird. Insbesondere eignen sich die Mineralsäuren Salz-, Schwefel- Salpeter- und Phosporsäure und die Halogenwasserstoffsäuren Fluor-, Chlor-, Brom- und Jodwasserstoffsäure.

Aluminiumoxid wird vorzugsweise in gekörnter Form eingesetzt. Dabei eignen sich besonders Korngrößen im Bereich von 0,05 bis 0,2 mm (70 bis 290 mesh).

Stereoisomerengemische von 8,12-Dihydroxy-13,14,15,16-tetranorlabdan sind literaturbekannt, z.B. aus der DE 32 40 054 A1. Erfindungsgemäß lassen sich aber auch beliebige andere Stereoisomerengemische von 8,12-Dihydroxy-13,14,15,16-tetranorlabdan zur Cyclisierung einsetzen. Die einsetzbaren Stereoisomerengemische können auch Nebenprodukte enthalten, wie sie im Zuge ihrer Herstellung angefallen sind. Beispielsweise stellen die gemäß DE 32 40 054 A1 (Beispiel 1 a-d) erhaltenen Diole komplexe Gemische stereoisomerer Alkohole dar, die zum Teil nicht näher identifiziert wurden. Derartige Gemische können auch Anteile aus den vorangehenden Reaktionsstufen enthalten; z. B. wurden NMR-spektroskopisch olefinische Protonen detektiert, die auf acyclische Strukturen hindeuten. Die in dem Diolgemischen enthaltenen Nebenprodukte wirken sich nicht nachteilig auf die geruchlichen Eigenschaften des Ambroxans aus, das nach dem erfindungsgemäßen Verfahren hergestellt wird. Gewünschtenfalls können die einzusetzenden Diolgemische zur Abtrennung von Nebenprodukten einer destillativen Reinigung unterworfen werden. Die Geruchs-Intensität des erfindungsgemäß hergestellten Ambroxans läßt sich durch solche Reinigungsoperationen in gewissen Grenzen modifizieren.

Die folgenden Beispiele sollen den Gegenstand der Erfindung erläutern und sind nicht einschränkend aufzufassen.

## Beispiele

### 1. Rohstoffe

Das für die Cyclisierung in Beispiel 1 eingesetzte Diolgemisch wurde in einer 5-stufigen Synthesesequenz hergestellt: Dazu wurde Nerolidol (Isomerengemisch) mit Phosphortribromid in Pyridin unter Umlagerung in Farnesylbromid (1) überführt, das mit Kaliumcyanid zum entsprechenden Nitril (2) umgesetzt wurde. Die Verseifung des Nitrils mit Kaliumhydroxid ergab Homofarnesylsäure (3), die mit Zinn-IV-chlorid zum Lactongemisch (4) cyclisiert wurde. Durch Reduktion von (4) mit Lithiumaluminiumhydrid wurde schließlich das Diolgemisch (5) erhalten. Die Schritte (2) bis (5) wurden in Anlehnung an Beispiel 1a-d) der DE 3240054 A1 durchgeführt.

**Saures Aluminiumoxid**: Handelsübliches mit HCl beladenes Aluminimoxid der Firma ICN Biomedicals. Der pH-Wert einer 5 Gew.-%-igen wäßrigen Suspension betrug 4,5.

### 2. Analytik

Das Diolgemisch (5) sowie die daraus gemäß Beispiel 1 durch Cyclisierung erhaltenen Stereoisomerengemische von Ambroxan wurden durch GC/MS-Kopplung analytisch charakterisiert. Dabei wurde im GC-Teil eine 25 m Quarzsäule (WG 11) mit einem Durchmesser von 0,2 μm verwendet. Für den MS-Teil wurde zur Ion-Trap-Detection ein Massenspektrometer ITD-Finnegan herangezogen. Die Erfassungsgrenze der Methode lag bei 0,2 %. Wegen der Verwendung einer nicht chiralen Säule beziehen sich alle Angaben auf racemische Mischungen.

### 3. Herstellvorschriften

### 3.1. Diolgemisch

(1) Farnesylbromid: 203,2 g Nerolidol wurden bei -8 bis -10 °C vorgelegt. Innerhalb von 4 Stunden wurde eine Mischung von 20,3 g Pyridin und 101,6 g Phosphortribromid zugetropft (exotherme Reaktion). Zur Aufarbeitung goß man die Lösung auf 400 ml kalte Natriumcarbonat-Lösung und extrahierte das entstandene Gemisch mit Diethylether. Die vereinigten organischen Phasen wurden nach dem Trocknen mit Natriumsulfat bei -30 °C aufbewahrt und erst unmittelbar vor der Weiterverarbeitung bei möglichst tiefer Temperatur eingeengt.

(2) Homofarnesylsäurenitril: 262,1 g Farnesylbromid, 91,2 g Kaliumcyanid, 150 ml Wasser und 7,7 g Tetrabutylammoniumchlorid wurden vorgelegt und die Mischung auf 60 °C erwärmt. Nach dem Anspringen der Reaktion wurde die Temperatur der Mischung durch Kühlung bei 80 °C gehalten. Nach Abklingen der Reaktion wurde zwei weitere Stunden bei 60 °C nachgerührt. Zur Aufarbeitung wurde die organische Phase abgetrennt, 2-mal mit je 200 ml Wasser extrahiert und über Natriumsulfat getrocknet. Das Produkt wurde über eine kurze Vigreux-Kolonne destilliert. Ausbeute: 108,7 g (52% d.Th. über die beiden ersten Stufen). IR-Spektrum: u.a. Bande bei 2200 $cm^{-1}$.

(3) Homofarnesylsäure: 108,7 g des Nitrils (2), 66,9 g Kaliumhydroxid, 600 ml Ethanol und 80 ml Wasser wurden vorgelegt und die Mischung 5 Stunden am Rückfluß gekocht. Anschließend wurde das Ethanol abdestilliert und der Rückstand in 2300 ml Wasser gelöst. Diese Lösung wurde 2-mal mit je 250 ml Ether extrahiert. Die organische Phase wurde verworfen. Die wäßrige Phase wurde mit 700 ml 20-%iger Schwefelsäure angesäuert und dann 3-mal mit je 250 ml Ether extrahiert. Die organischen Phasen wurden vereinigt, mit Wasser gewaschen, über Natriumsulfat getrocknet und eingeengt. Ausbeute: 97,5 g eines gelben Öls (83 % d.Th.).

(4) Lactongemisch: 79,5 g Homofarnesylsäure wurden in 480 ml wasserfreiem Methylenchlorid vorgelegt und auf -78 °C gekühlt. Innerhalb einer Stunde wurden 100 g Zinn-IV-chlorid zugetropft. Anschließend wurde 2 Stunden bei -78 °C nachgerührt. Zur Aufarbeitung wurde das Gemisch auf 600 ml Wasser gegossen und die organische Phase abgetrennt. Man wusch die organische Lösung mit gesättigter Natriumcarbonat-Lösung und mit Wasser, trocknete über Natriumsulfat und destillierte das Lösungsmittel ab. Dabei fiel das Lactongemisch in quantitativer Ausbeute an.

(5) <u>Diolgemisch</u>: Zu 24,1 g Lithiumaluminiumhydrid in 200 ml wasserfreiem Diethylether wurden bei Raumtemperatur 79,5 g des Lactongemisches (4) langsam unter Rühren zugetropft (exotherme Reaktion). Nach vollständiger Zugabe wurde eine weitere Stunde am Rückfluß nachgerührt. Zur Aufarbeitung kühlte man das Gemisch ab und tropfte 100 ml einer 10%-igen Natronlauge zu. Anschließend trennte man die organische Phase ab und extrahierte die wäßrige Phase mit Diethylether. Die vereinigten organischen Phasen wurden mit 10%-iger Natriumsulfatlösung neutral gewaschen, über Natriumsulfat getrocknet und eingeengt. Ausbeute: 75,9 g Diol (94 % d.Th.). In dem so erhaltenen Diolgemisch wurden durch GC/MS Analyse nachgewiesen:

| | |
|---|---|
| Diovorstufe des Ambroxans: | 31,2 % |
| Diolvorstufe des 9-epi-Isomers des Ambroxans: | 19,8 % |
| Diolvorstufe des 8-epi-Isomers des Ambroxans: | 2,0 % |
| | 53,0 % |

## 3.2. Beispiel 1: Ambroxangemisch

130 g des Diolgemisches (5) wurden in 200 ml Toluol gelöst. Die Lösung wurde mit 102 g saurem Aluminiumoxid versetzt und unter Rühren zum Sieden erhitzt. Am Wasserabscheider wurde eine Wasserabscheidung beobachtet, die nach einer Stunde zum Stillstand kam. Nun wurde die Temperatur durch Abnahme von Toluol aus dem Wasserabscheider auf 150 °C eingestellt. Dabei erfolgte eine weitere kontinuierliche Wasserauskreisung. Als diese nach ca. 4 Stunden beendet war, wurde (noch heiß) vom Aluminiumoxid abfiltriert und mit **50 ml** heißem Toluol nachgespült. Es wurden 123 g Rohprodukt erhalten. Die Untersuchung des Produktes mittels GC/MS ergab:

| | |
|---|---|
| Ambroxan: | 30,8 % |
| 9-epi-Isomer des Ambroxans: | 19,4 % |
| 8-epi-Isomer des Ambroxans: | 2,0 % |
| | 52,2 % |

Daraus ergibt sich für die Cyclisierung eine Ausbeute von 98,5 % der Theorie.

## Patentansprüche

1. Verfahren zur Herstellung von Stereoisomerengemischen von 8,12-Oxido-13,14,15,16-tetranorlabdan durch cyclisierende Dehydratisierung von Stereoisomerengemischen von 8,12-Dihydroxy-13,14,15,16-tetranorlabdan, dadurch gekennzeichnet, daß man zur Cyclisierung säurebeladenes Aluminiumoxid verwendet.

2. Verfahren gemäß Anspruch 1, dadurch gekennzeichnet, daß man die Cyclisierung bei Temperaturen von 120 bis 180, vorzugsweise 140 bis 160 °C durchführt.

3. Verfahren gemäß einem der Ansprüche 1 bis 2, dadurch gekennzeichnet, daß man die Cyclisierung in einem inerten Lösungsmittel durchführt.

4. Verfahren gemäß einem der Ansprüche 1 bis 3, dadurch gekennzeichnet, daß man das säurebeladene Aluminiumoxid - bezogen auf das 8,12-Dihydroxy-13,14,15,16-tetranorlabdan - in einer Menge von 40 bis 100 Gew.-%, vorzugsweise 60 bis 80 Gew.-% einsetzt.

## Claims

1. A process for the production stereoisomer mixtures of 8,12-oxido-13,14,15,16-tetranorlabdane by cyclizing dehydration of stereoisomer mixtures of 8,12-dihydroxy-13,14, 15,16-tetranorlabdane, characterized in that acid-treated aluminium oxide is used for the cyclization.

2. A process as claimed in claim 1, characterized in that the cyclization is carried out at temperatures of 120 to 180°C and preferably at temperatures of 140 to 160°C.

3. A process as claimed in claim 1 or 2, characterized in that the cyclization is carried out in an inert solvent.

4. A process as claimed in any of claims 1 to 3, characterized in that the acid-treated aluminium oxide is used in a quantity of 40 to 100% by weight and preferably in a quantity of 60 to 80% by weight, based on the 8,12-dihydroxy-13,14,15,16-tetranorlabdane.

**Revendications**

1. Procédé d'obtention de mélanges de stéréoisomères de 8,12-oxydo-13,14,15,16-tétranorlabdane par déshydratation cyclisante de mélanges de stéréoisomères de 8,12-dihydroxy-13,14,15,16-tétranorlabdane, caractérisé en ce que l'on utilise pour la cyclisation de l'oxyde d'aluminium chargé avec de l'acide.

2. Procédé selon la revendication 1, caractérisé en ce que l'on effectue la cyclisation à des températures de 120 à 180°C, de préférence de 140 à 160°C.

3. Procédé selon l'une des revendications 1 à 2, caractérisé en ce que l'on effectue la cyclisation dans un solvant inerte.

4. Procédé selon l'une des revendications 1 à 3, caractérisé en ce que l'on met en oeuvre l'oxyde d'aluminium chargé avec de l'acide, rapporté au 8,12-dihydroxy-13,14,15,16-tétranorlabdane, en quantité allant de 40 à 100 % en poids, de préférence de 60 à 80 % en poids.